(19) Europäisches Patentamt
European Patent Office
Office européen des brevets

(11) EP 0 728 517 B1

(12) EUROPÄISCHE PATENTSCHRIFT

(45) Veröffentlichungstag und Bekanntmachung des Hinweises auf die Patenterteilung:
**23.09.1998 Patentblatt 1998/39**

(51) Int. Cl.[6]: **B01D 61/44**, C07D 307/33, C07C 67/02, C07C 69/72

(21) Anmeldenummer: **95120333.0**

(22) Anmeldetag: **21.12.1995**

(54) **Verfahren zur Herstellung von Ketoverbindungen**

Process for preparing keto compounds

Procédé de préparation de composés cétoniques

(84) Benannte Vertragsstaaten:
**AT BE CH DE ES FR IT LI LU NL PT**

(30) Priorität: **21.02.1995 DE 19505957**

(43) Veröffentlichungstag der Anmeldung:
**28.08.1996 Patentblatt 1996/35**

(73) Patentinhaber:
**HÜLS AKTIENGESELLSCHAFT**
**45764 Marl (DE)**

(72) Erfinder:
**Sridhar, Srinivasan, Dr.**
**D-45770 Marl (DE)**

(56) Entgegenhaltungen:
**EP-A- 0 702 998** **GB-A- 681 196**

- **"The chemistry of the carbonyl group" 1966 , PATAI, S. , LONDON, GB XP002020903 * Seite 273, Absatz 2 - Seite 276, Absatz 1 ***

EP 0 728 517 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft ein neues Verfahren zur Herstellung von Ketoverbindungen durch Kondensationsreaktionen.

Ketoverbindungen sind wertvolle Zwischenprodukte bei der Herstellung von z. B. Heterocyclen, Pharmazeutika, Pflanzenschutzmitteln und Aromastoffen.

Durch Esterkondensationen können Carbonsäureester zu Dicarbonylverbindungen umgesetzt werden. Nach dieser Methode kann beispielsweise Acetessigester aus Essigester hergestellt werden, wie z. B. beschrieben in "The chemistry of the carbonyl group", Patai S., Interscience, London 1966, Seiten 273-276 und GB-A-0 681 196. Ebenfalls können auch Lactone unter Einsatz von Essigester in die entsprechenden Acetylderivate übergeführt werden. Dabei erfolgt die Reaktion in zwei Schritten. Im 1. Schritt wird eine Kondensation unter alkalischen Bedingungen vorgenommen, worauf das entstandene Enolat im 2. Schritt protoniert wird. Die Reaktion wird am Beispiel der Herstellung von Acetessigsäuremethylester aus Essigsäuremethylester durch folgendes Schema verdeutlicht:

1. Kondensation

$$2\ CH_3COOCH_3 + CH_3ONa \longrightarrow CH_3C{=}CHCOOCH_3 + 2\ CH_3OH$$

|

$O^-)Na^+$

Essigsäuremethylester Enolat

2. Protonierung

$$CH_3C{=}CHCOOCH_3 + H^+ \longrightarrow CH_3COCH_2COOCH_3 + Na^+$$

|

$O^-)Na^+$

Enolat Acetessigsäuremethylester

Nach dem Verfahren der alteren nicht vorveröffentlichten EP-A-0 702 998, übereinstimmend mit der DE-A-4 433 823 wird der 2. Schritt, die Protonierung, elektrodialytisch durchgeführt. Bei der Protonierung verwendet man hier vorzugsweise einen Alkohol, der dabei in ein Alkalialkoholat übergeführt wird. Der Alkohol wirkt also als Protonendonator, der gleichzeitig Alkaliionen abfängt.

Aufgabe der vorliegenden Erfindung war es, das Verfahren von EP-A-0 702 998 unter Beibehaltung der Vorteile - Wiedergewinnung des Kondensationsmittels, Vermeidung einer systemfremden Säure und Vermeidung eines Salzanfalls - noch einfacher zu gestalten.

Die Aufgabe wird erfindungsgemäß dadurch gelost, daß man sowohl die Kondensation als auch die Protonierung in einem Verfahrensschritt elektrodialytisch durchführt.

Die Ketoverbindungen, die erfindungsgemäß hergestellt werden, können einfache Ketone, Diketone, Ketoester, -halogenide, -aminosäuren oder -nitroverbindungen sein. Vorzugsweise handelt es sich um Ketoester und im besonderen um β-Ketoester.

Die Kondensation erfolgt im allgemeinen in Gegenwart von Alkalialkoholaten. Geeignete Alkaliionen sind dabei vorzugsweise das Natrium- und das Kaliumion.

Die Alkoholate leiten sich meist von den Alkoholen ab, die auch als Lösemittel verwendet werden. Geeignet sind beispielsweise Alkohole mit 1 bis 5 C-Atomen, wie Methanol, Ethanol, Isopropanol oder tert.-Butanol, Diole, wie Glykol und Butandiol, oder Triole, wie Glycerin. Es können auch Alkoholmischungen eingesetzt werden. Die Alkohole können bis zu 5 % Wasser enthalten. Sie können auch ein Edukt oder ein Produkt enthalten.

Die Elektrodialyse wird vorzugsweise in einer elektrodialytischen Zelle mit 2n + 2 Kammern, wobei n eine Zahl von 1 bis 60 ist, durchgeführt. In besonders bevorzugten Fällen ist n eine Zahl von 1 bis 10.

Die Elektrodialyse erfolgt vorzugsweise bei -20 bis +90 °C. Dabei werden Temperaturen von 10 bis 50 °C ganz besonders bevorzugt.

Durch die Kombination von Kondensation und Protonierung in einem Verfahrensschritt wird das bei der Kondensation verbrauchte Alkoholat während der Protonierung zurückgewonnen. Alkoholat kann dabei im Kreise geführt werden. Das Verfahren von EP-A-0 702 998 wird unter Beibehaltung der dort genannten Vorteile weiter vereinfacht, da nun auch die Kondensation in den elektrodialytischen Prozeß einbezogen wird.

Die Reaktion wird nachfolgend am Beispiel der Kondensation von Essigsäuremethylester zu Acetessigsäuremethylester dargestellt. Dabei ist das vorliegende Verfahren nicht auf diese Reaktion begrenzt.

Die Vorgehensweise wird am Beispiel einer elektrodialytischen Zelle, bestehend aus 6 Kammern, gemäß Abbildung 1 erläutert: Die Kammern werden jeweils durch eine Kationenaustauschmembran K und eine bipolare Membran B begrenzt. Anstelle der bipolaren Membran kann auch ein Membranpaar, bestehend aus einer Anionen- und einer Kationenaustauschmembran, verwendet werden.

In die Anodenkammer III wird eine methanolische Lösung von Natriumenolat des Acetessigsäuremethylesters eingeführt. Dieser Anolyt bildet einen getrennten Kreislauf. In die Kammern I wird ein Gemisch aus Essigsäuremethylester und Methanol eingeführt. Das Gemisch wird "Konzentrat" genannt. Die Kammern II werden mit einer methanolischen Lösung des Natriumenolats des Acetessigsäuremethylesters beschickt. Dieses Gemisch wird "Diluat" genannt.

Beim Anlegen eines elektrischen Feldes wandern die Alkaliionen aus der Anodenkammer sowie aus den Kammern II durch die Kationenaustauschmembranen K in Richtung der Kathode in die Kammern I und bilden dort das Alkoholat. Die Entstehung des Kondensationsmittels führt zur Kondensation des Carbonsäureesters in diesen Kammern. Die Kammern I stellen also einen Reaktorraum dar, in dem der Acetessigsäuremethylester in der Enolat-Form entsteht. Durch die bipolaren Membranen B treten Protonen. Deshalb erfolgt in den Kammern II die Protonierung des Enolats, wodurch der freie Acetessigsäuremethylester entsteht.

Die Kondensation ist eine Gleichgewichtsreaktion und erfordert eine laufende Abtrennung des entstehenden Alkohols. Beispielsweise wird das Konzentrat solange elektrodialytisch behandelt, bis die Kondensation das Gleichgewicht fast erreicht hat. Anschließend wird es in einen externen als Reaktor wirkenden Behälter (nicht in Abbildung 1 angegeben) geführt. Die Reaktion wird dann unter destillativer Abtrennung des Alkohols mittels eines Schleppmittels fortgeführt. Der verbleibende, nicht umgesetzte Essigsäureester wird als Ausgangsstoff für die nächste Charge eingesetzt.

In einer besonderen Ausführung des erfindungsgemäßen Verfahrens hält man den Konzentratbehälter bei einer erhöhten Temperatur, um die Kondensation zu beschleunigen. Die oben genannte Destillation kann dann direkt aus dem Konzentratbehälter mit einer aufgesetzten Kolonne erfolgen. Durch eine derartige Ausführung können Kondensation und Protonierung zügig durchgeführt und aufeinander abgestimmt werden.

Nach einer absatzweisen Elektrodialyse wird die Produktlösung aus dem Diluatbehälter abgelassen und der Behälter mit dem frisch entstandenen Kondensationsprodukt (noch in Form des Na-Enolats) aus dem Konzentrationsbehälter gefüllt. Das verbrauchte Anolyt wird durch den entsprechenden Anteil aus dem abgelassenen Diluat ersetzt. Auf diese Weise werden die Natriumionen im System erhalten. Die Nettogleichung des Verfahrensschritts entspricht:

$$2\ CH_3COOCH_3 \rightarrow CH_3COCH_2COOCH_3 + CH_3OH$$

Der größere Rest des Diluats wird einer Destillationsstufe zugeführt, um den Acetessigsäureester von Methanol zu befreien, sofern dies nicht bereits im Konzentrationsbehälter erfolgte. Nach Befüllen des Konzentrationsbehälters mit frischer Essigsäuremethylesterlösung kann die nächste absatzweise Umsetzung begonnen werden.

Weil die Kondensation eine Gleichgewichtsreaktion ist, versucht man, die entstehenden Produkte laufend aus dem System abzutrennen. In einer weiteren besonderen Ausführung des erfindungsgemäßen Verfahrens führt man den Konzentratstrom bereits nach einem geringfügigen Umsatz in der Elektrodialyseeinheit gleich in die Diluatkammern II ein. Hier erfolgt die Protonierung der gerade entstandenen geringen Menge an Enolat. Dadurch wird das Enolat aus dem chemischen Gleichgewicht der Kondensation abgeführt. Der Diluatstrom wird dann einer Destillation unterworfen, um auch den entstandenen Alkohol abzutrennen. Anschließend wird die Lösung wieder dem Konzentratbehälter zurückgeführt, um die Reaktion fortzusetzen. Bei dieser Ausführung erfolgt eine ständige Kondensation und Protonierung im Kreislauf.

Des weiteren kann man nach dem erfindungsgemäßen Verfahren kondensieren und gleichzeitig umestern. Dieses ist dann möglich, wenn der Alkohol des Konzentrats von dem Alkohol des Diluats verschieden ist. So kann man beispielsweise aus Essigsäuremethylester den Acetessigsäureethylester herstellen.

Das folgende Beispiel soll die Erfindung verdeutlichen.

## Beispiel 1

Entsprechend der bereits erfolgten Beschreibung wird ein Stapel verwendet, der mit vier Kreisläufen, nämlich zwei Elektrodenkreisläufen, einem Diluat- und einem Konzentratkreislauf versehen ist. Die Membranen stammen von

TOKUYAMA SODA Corp. Anstelle einer Bipolarmembran wird ein Membranpaar bestehend aus einer Anionen- und einer Kationenaustauschmembran eingesetzt. Die Reihenfolge der Membranen ist von der Anode zur Kathode wie folgt:
eine Kationenaustauschmembran (Typ C66-10F), ein Membranpaar (Typ AM 1 und CM 1) und wieder eine Kationenaustauschmembran (Typ C66-10F), wonach die Kathode folgt. Die effektive Membranfläche je Membran entspricht 100 $cm^2$. Der Versuch verläuft bei Normaltemperatur. Nur der Konzentratkreislauf, in dem die Kondensation erfolgt, wird bei 45 °C gehalten.

Als Anolyt werden 1 388 g einer Lösung von 6 Gewichtsprozent Natriummethanolat in Methanol vorgelegt. Der Katholyt besteht anfangs aus einer 1,23gewichtsprozentigen Lösung von Natriummethanolat in Methanol. Als Diluat (Protonierkreislauf) dienen 1 282 g einer Lösung von 10 Gewichtsprozent Natriumenolat des Acetessigsäuremethylesters in Methanol. Als Konzentrat (Reaktionskreislauf) werden 1 450 g einer Lösung von 15,78 Gewichtsprozent Methylacetat eingesetzt. Die Lösung enthält ferner 0,5 Gewichtsprozent Natriummethanolat als Leitsalz.

Die Lösungen werden in entsprechenden Kreisläufen ständig umgepumpt. Zwischen den Elektroden wird eine Spannung von 120 V angelegt. Nach 26 Stunden und 40 Minuten wird der Versuch abgebrochen, worauf die Proben aus den Kreisläufen analysiert werden. Es werden im Konzentratkreislauf 12,4 g Na-Enolat des Acetessigesters produziert. In der gleichen Dauer werden im Diluatkreislauf 11,7 g freier Acetessigester hergestellt.

Aus den Ergebnissen geht hervor, daß eine erfindungsgemäße gleichzeitige Kondensation und Protonierung möglich ist.

## Patentansprüche

1. Verfahren zur Herstellung von Ketoverbindungen durch Kondensationsreaktionen,
dadurch gekennzeichnet,
daß man die Kondensation und die Protonierung in einem Verfahrensschritt elektrodialytisch durchführt.

2. Verfahren nach Anspruch 1,
dadurch gekennzeichnet,
daß Ketoester hergestellt werden.

3. Verfahren nach Anspruch 1,
dadurch gekennzeichnet,
daß man Kondensation und Protonierung in einer elektrodialytischen Zelle mit 2n + 2 Kammern, wobei n eine Zahl von 1 bis 60 ist, durchführt.

4. Verfahren nach Anspruch 3,
dadurch gekennzeichnet,
daß n eine Zahl von 1 bis 10 ist.

5. Verfahren nach Anspruch 1,
dadurch gekennzeichnet,
daß man Kondensation und Protonierung bei -20 bis +90 °C, vorzugsweise bei 10 bis 50 °C, durchführt.

6. Verfahren nach Anspruch 2,
dadurch gekennzeichnet,
daß man gleichzeitig elektrodialytisch eine Umesterung durchführt.

## Claims

1. A process for the preparation of keto compounds by condensation reactions, characterized in that the condensation and the protonation are carried out in one process step by electrodialysis.

2. A process according to claim 1, characterized in that keto esters are prepared.

3. A process according to claim 1, characterized in that condensation and protonation are carried out in an electrodialysis cell having 2n + 2 chambers, where n is a number from 1 to 60.

4. A process according to claim 3, characterized in that n is a number from 1 to 10.

5. A process according to claim 1, characterized in that condensation and protonation are carried out at from -20 to +90°C, preferably at from 10 to 50°C.

6. A process according to claim 2, characterized in that a transesterification is carried out simultaneously by electrodialysis.

**Revendications**

1. Procédé de fabrication de composés cétoniques par des réactions de condensation,
caractérisé en ce qu'
on réalise la condensation et la protonation dans une étape de procédé par voie électrodialytique.

2. Procédé selon la revendication 1,
caractérisé en ce que
sont fabriqués des esters cétoniques.

3. Procédé selon la revendication 1,
caractérisé en ce qu'
on réalise la condensation et la protonation dans une cellule électrodialytique ayant 2n + 2 chambres, dans laquelle n est un nombre de 1 à 60.

4. Procédé selon la revendication 3,
caractérisé en ce que
n est un nombre de 1 à 10.

5. Procédé selon la revendication 1,
caractérisé en ce qu'
on réalise la condensation et la protonation entre -20 et +90°C, de préférence entre 10 et 50°C.

6. Procédé selon la revendication 2,
caractérisé en ce qu'
on réalise en même temps une transestérification par voie électrodialytique.

Abb. 1

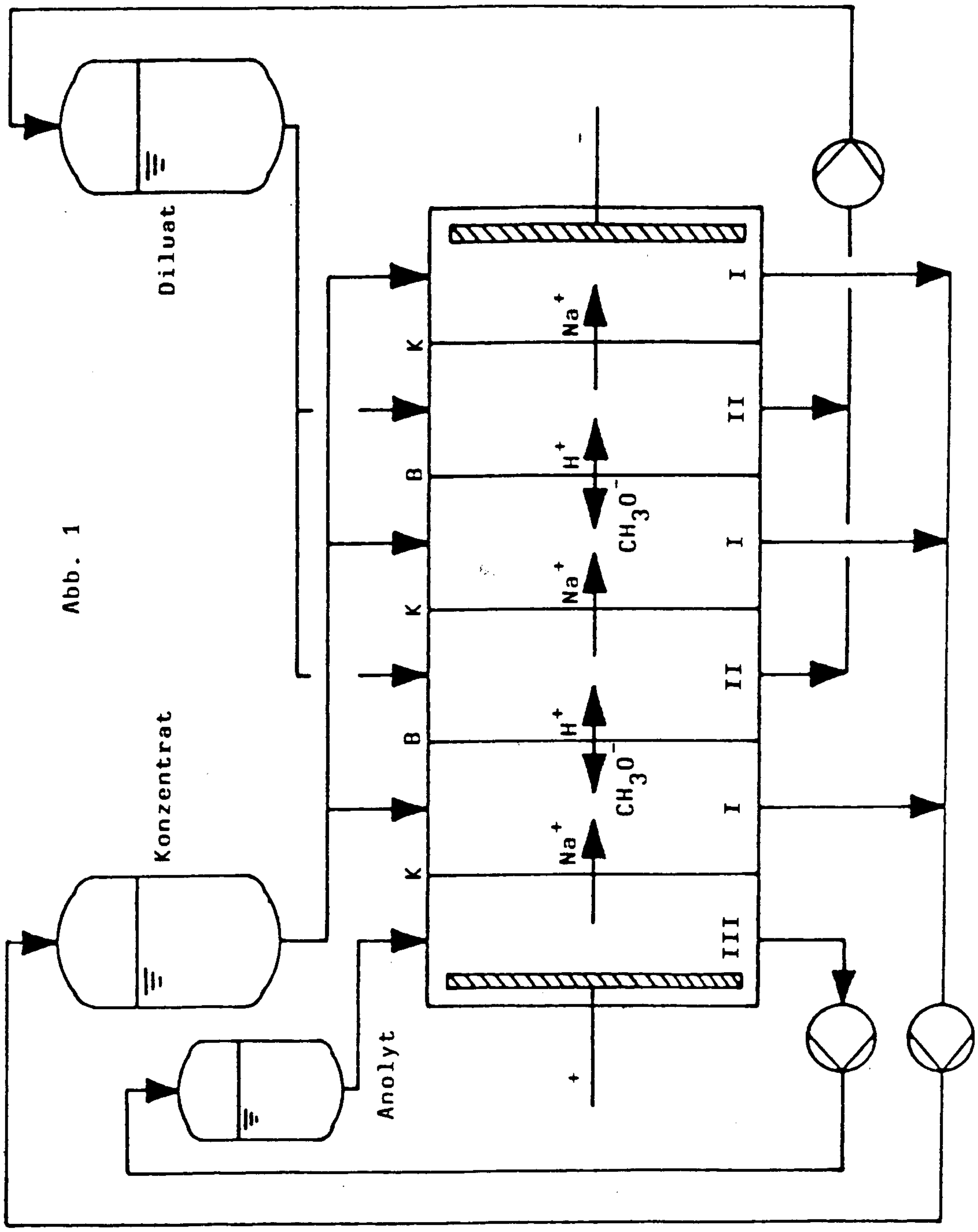
Diluat
Konzentrat
Anolyt
+
-
K
B
K
B
K
I
II
I
II
I
III
Na+
H+
CH3O-
Na+
H+
CH3O-
Na+